# EUROPEAN PATENT SPECIFICATION

(11) **EP 4 324 616 B1**
(45) Date of publication and mention of the grant of the patent: **19.08.2026**
(21) Application number: 24150036.2
(22) Date of filing: 22.01.2019
(51) Int. Cl.: A61M 25/00, B29C 65/02, B29C 45/14, B29L 31/00

(54) **A METHOD OF MANUFACURING A URINARY CATHETER HAVING AN INJECTION MOLDED TIP AND THE URINARY CATHETER**
VERFAHREN ZUR HERSTELLUNG EINES HARNKATHETERS MIT SPRITZGEGOSSENER SPITZE UND EIN HARNKATHETER
PROCEDE DE FABRICATION D'UN CATHÉTER URINAIRE À POINTE MOULÉE PAR INJECTION ET UN CATHÉTER URINAIRE

(43) Date of publication of application: 21.02.2024
(62) Document number(s) of the earlier application(s) in accordance with Art. 76 EPC: 19153049.2 / 3 685 872
(73) Proprietor: Wellspect AB, 431 21 Mölndal (SE)
(72) Inventor: UTAS, Jan, 434 95 Kungsbacka (SE); DAHLBERG, Niklas, 443 38 Lerum (SE)
(74) Representative: Allen, Caroline Margaret

(56) References cited:
- EP-A1- 3 040 097
- EP-A2- 0 778 045
- WO-A1-2014/052770
- WO-A1-2015/160492
- AU-B2- 503 664
- US-A- 5 360 330
- US-A1- 2005 182 354
- US-A1- 2006 142 733
- US-A1- 2008 108 978
- US-A1- 2009 043 287

## Description

### Field of the invention

The present invention generally relates to a urinary catheter comprising a tubular shaft extending between an insertable end and a discharge end, and having a tip fixedly connected to the insertable end of the tubular shaft. The invention is also related to a corresponding method of manufacture of such urinary catheters.

### Background of the invention

The present invention relates to urinary catheters, and in particular urinary hydrophilic catheters. Urinary catheters are commonly used for draining urine from the bladder. One type of urinary catheters is indwelling catheters, so-called Foley catheters, which are maintained in place in the urethra for an extended period of time, such as for days, weeks or even months. Another type of urinary catheters are intended for short term use, so-called intermittent catheters. Intermittent urinary catheters are used for draining the bladder once, and then be removed. Intermittent catheters are typically used for a few minutes, and catheterization is typically made by the user him/her self, so-called self-catheterization, and is typically performed many times a day. In order to maintain the catheter in a clean and preferably sterile condition, each catheter is normally pre-packed in a receptacle by the manufacturer, thereby providing a catheter assembly. Typically catheters for intermittent catheterization are used by patients suffering from urinary incontinence or by disabled individuals like para- or tetraplegics. Using an intermittent catheter, the bladder may be drained through a natural or artificial urinary canal. Many catheters for intermittent catheterization are provided with a hydrophilic coating or the like, providing a smooth and slippery surface when wetted, for safe and comfortable insertion in the urinary canal.

For urinary catheters, certain parameters need to be satisfied by the material from which the elongate shaft is manufactured. The material must fulfill such requirements as softness, good kink resistance, good dimensional stability, minimal shrinkage, processability, for example ease to form and glue, and the possibility to be sterilized by radiation, steam, ethylene oxide or other means. For some products, there is further the need for the material to accept a surface treatment which will impart desired surface properties to the medical device, such as hydrophilicity. To this latter end, the chemistry of the substrate material is of importance since this affects the possibility to coat the substrate.

However, insertion of a urinary catheter into the urethra is often cumbersome, and associated with certain risks. This is particularly the case for male catheters. A male catheter is relatively long, typically 35-40 cm, to be able to extend through the whole length of the urethra. The insertable length of the catheter is normally 200-350 mm for male users. The male urethra is also curved in many places, and comprises sections with reduced cross-sections. Thus, during the introduction of the catheter into the urethra and while guiding the catheter tip through the urethra into the bladder, it is necessary to overcome pockets, folds, bends, strictures, enlarged prostate and/or the like. If one pushes the catheter with a corresponding force against the existing impediments in the urethra, one will face a considerable risk of injury.

To overcome such insertion problems, it is known to use special tips on the catheter. For example, the catheter may be provided with a curved tip, often referred to as a Tiemann or Coudé type catheter. Tiemann and Coudé catheters have a tip which is angled upward, to assist in negotiating the male prostatic curve. Thus, this tip form facilitates passage through the bladder neck in the presence of obstruction e.g. from a slightly enlarged prostate gland (e.g. in benign prostatic hyperplasia), and can be helpful for such and ofther difficult insertions. Such tips are e.g. known from EP 0 799 069 by the same applicant.

Other examples of specifically designed tips are found in EP 0 384 476, US 2004/193143 and US 2016/184551.

However the production of such specifically designed tips are often problematic. The tips may be formed directly from the shaft material. For example, a very common way to make such tips is to extrude tubing from a thermoplastic polymer material, and cut it to the desired length. One of the cut ends is then formed into a tip shape in a secondary operation, for example, by forcing the cut end into a heated die that has the desired tip shape. However, shaping and forming the material into a desired shape and with desired properties is cumbersome and costly, if at all possible. Further, it may be difficult to coat such catheters, e.g. with a hydrophilic coating.

US 2006/0142733, WO 2015/160492 AU 503664 and US 2005/0182354 disclose other methods for forming an injection molded tip on an end of a catheter shaft.

WO 2014/052770 discloses a method for forming a catheter shaft, including a closed end, by injection molding.

US 2009/0043287 is directed a catheter movement system, allowing the catheter to be gripped through the walls of an outer pouch, or to prevent backward movement of the catheter when expelled out from the pouch through an opening.

US 2008/0108978 discloses a urinary retention catheter, with an expandable retention element made by a woven mesh, arranged adjacent to, or overlying, an insertion tip of the catheter.

US 5360330 discloses a method for thermoforming of a closed end in a catheter shaft.

EP 0778045 discloses a method for thermoforming an open end of an intravenous catheter.

Further, the tip may be formed separately, and be attached to the shaft by adhesion or the like. Various attempts to connect tips are e.g. disclosed in US 2007/0035989, US 5835518, US 2006/0167421, US 5762637 and US 6149996. However, even though this allows for a great freedom in chosing the desired shape and properties of the tip, the connection to the shaft is cumbersome and costly, and there is also a risk that the tip is inadequately connected, leading to a risk that it may fall off during use. Should a tip fall off inside the user, this may lead to fatal consequences.

Thus, there is still a need for urinary catheters where a separately formed tip may be connected to a catheter shaft in a simpler and more cost-effective way, and still ensure that the tip is securely fixed to the shaft.

### Summary of the invention

It is a general object of the present invention to alleviate the above-discussed problems.

This problem is solved by means of a urinary catheter and a method for producing a urinary catheter in accordance with the present invention, as defined in the appended claims.

According to a first aspect of the disclosure (not claimed) there is provided a method for producing a urinary catheter, comprising the steps:
providing a tubular catheter shaft having an axially extending lumen therein;
thermoforming an end of the tubular catheter shaft into a closed end;
inserting said closed end of the tubular catheter shaft into an injection mold cavity; and
injecting thermoplastic polymer material into the mold cavity to form a tip on the thermoformed end of the tubular catheter shaft.

According to this method, an end of the tubular shaft can be closed by thermoforming, in a per se known way. This is a simple procedure, and is made even more simple since there is no need to form any specific shape and finish of the end. This is due to the fact that the end is anyway to be overmolded by the tip in the subsequent tip forming step. Thus, the closed end of the tubular catheter shaft will simply serve as a connection surface for the tip, and will in the finished catheter only exist interiorly of the catheter, covered by the tip. Further, the thermoformed closed end ensures that the injected material to form the tip does not enter into the lumen of the tubular shaft.

According to a first aspect of the invention there is provided a method for producing a urinary catheter, comprising the steps:
providing a tubular catheter shaft having an axially extending lumen therein;
inserting an end of the tubular catheter shaft with a non-closed opening into said lumen into an injection mold cavity; and
injecting thermoplastic polymer material into the mold cavity to form a tip on the end of the tubular catheter shaft, whereby the injecting is controlled so that a limited amount of thermoplastic polymer enters into the lumen.

This method is even simpler than the first discussed method, since it does not require any thermoforming of the tip. In this method, the tubular shaft, including an opening into the lumen, is arranged in the mold cavity. It has been found by the present inventors that by controlling the injection molding parameters, such as one or several of injection speed, injection time, and volume of injected material, it is possible to control how deep into the lumen the injected material will penetrate. Hereby, the injected material forms a controlled stub shaft protruding into the lumen, thereby forming a strong fixation to the shaft.

In order to further control the depth of introduction of the thermoplastic material into the lumen, it is further feasible to provide an overpressure within the axially extending lumen during the injecting of the thermoplastic polymer material into the mold cavity. This overpressure may then be controlled to provide a resistance for the thermoplastic polymer material to enter into the lumen, and the overpressure may be controlled to provide an adequate length of penetration. Providing a higher overpressure will provide a stronger resistance and a lower depth of penetration, whereas a lower overpressure, or no overpressure at all, will provide less resistance and a corresponding deeper penetration.

In both the above-discussed methods, a very strong bond between the shaft and the injection molded tip is created, inter alia due to the large overlapping areas.

The mold cavity may further be heated during molding, whereby the material of the shaft will be softened not only by the heated injection molded thermoplastic polymer material, but also by the heating of the mold. This ensures an even stronger attachment of the tip to the shaft, since the materials will hereby also be fused together. However, the heat provided by the heated injected thermoplastic polymer material is often sufficient to provide sufficient heating to form a strong attachment, and in such cases, no additional heating of the mold is required.

In these methods, the forming of the tip may further comprise cooling of the mold cavity to solidify the injected thermoplastic polymer material, and removing the tubular catheter shaft with the formed tip from the mold. Cooling of the mold, after having introduced the thermoplastic polymer material, and thereby having formed the tip, ensures the integrity of the shaped tip, and also facilitates removal from the mold cavity.

The methods may further comprise the step of coating at least a part of the tubular shaft and the tip with a hydrophilic coating. The coating of the tubular shaft together with the tip can, advantageously, be made after formation of the tip. However, alternatively, the shaft may be coated prior to the injection molding, in which case coating only of the tip, and perhaps a short length of the shaft in the vicinity of the tip, need to be coated after the injection molding.

The mold cavity may be arranged to provide a desired surface texture on the injection molded tip, and e.g. a surface texture corresponding to the surface texture on the shaft. Hereby, coating of the tip is facilitated.

The methods may further comprise the step of inserting an elongate insert into the axially extending lumen prior to inserting an end of the catheter shaft into the mold, wherein the insert has a smaller outer diameter than the inner diameter of the lumen, forming a gap between an outer circumferential surface of the elongate insert and an inner circumferential surface of the lumen, the insert thereby being loosely arranged within the lumen. The elongate insert may e.g. be a narrow steel rod or the like. Insertion of such an elongate insert does not affect the penetration of the injected thermoplastic polymer material, but ensures that the shape of the catheter shaft is not altered during the injection molding process. For example, it prevents inadvertent collapsing of the shaft. Thus, the elongate insert ensures that the shaft remain straight, and with an equally sized lumen along its length. However, in many cases, depending on the materials used and the process parameters, such as the temperature, there is no need for such an additional precaution, and in such cases there is no need for use of such an elongate insert.

Preferably, the thermoplastic polymer is heated to a temperature exceeding the melting temperature of the tubular catheter shaft immediately prior to being injection molded in to the mold cavity, and preferably exceeding said melting temperature with at least 10 degrees C. Hereby, it has been found, a very strong adhesion between the shaft and the tip is obtained, without any risk of damage and deformation of the shaft.

The tip may have an outer diameter which at all places is equal to or lower than the outer diameter of the tubular shaft. However, alternatively, the tip may have a diameter which at least on some positions has a diameter exceeding the diameter of the shaft. For example, the tip may form a ball shape at the end of the catheter.

The urinary catheter is preferably an intermittent urinary catheter, intended for short time use, such as catheterization of a few minutes duration, being repeated a number of times each day. The term "short term use" indicates a use that is limited in time, and in particular limited to a time period of less than 15 minutes, and preferably less than 10 minutes, and most preferably less than 5 minutes.

The urinary catheter is also preferably a male catheter, preferably having a total length within the range of 35-40 cm, and having an insertable length within the range of 200-350 mm.

According to a second aspect of the invention, there is provided a urinary catheter comprising a tubular shaft extending between an insertable end and a discharge end, the insertable end being thermoformed to form a closed end, and a tip fixedly connected to said closed end of the tubular shaft.

Such a catheter may, as discussed in the foregoing, be produced very cost-effectively, and at the same time ensuring a very strong connection between the tip and the catheter shaft. The tip may also be shaped into any desired shape, and may also have tailored properties, for example in relation to hardness, rigidity, etc.

For example, the hardness of the tip may be equal to or lower than 60 micro Shore A, and preferably equal to or lower than 50 micro Shore A, and most preferably equal to or lower than 45 micro Shore A. In some embodiments, the hardness of the tip may be significantly lower than the hardness of the shaft.

Further, the tip may have a hardness which is equal to or higher than 20 micro Shore A.

In embodiments, the tip may have a lower micro Shore A hardness than the tubular shaft, and preferably a micro Shore A hardness that is at least 10% lower, and more preferred at least 30% lower, and most preferably at least 50% lower.

The thermoformed end preferably forms a tapered contacting surfaces for the tip. Hereby, a larger attachment area, and a stronger attachment, is obtained.

The tip and the tubular shaft may be formed of different materials. This facilitates tailoring of the requested properties of the tip. However, alternatively, the tip and the shaft can be made of the same material.

Preferably, the difference between a melting temperature of the material of the tip and a melting temperature of the material of the tubular shaft is less than 20 degrees C, and preferably less than 15 degree C, and most preferably less than 10 degree C. Further, it is preferred that the melting temperature of the material of the tubular shaft is higher than the melting temperature of the material of the tip. A relative closeness in melting temperatures ensures that a very strong connection is formed between the tip and the shaft. Provision of a higher melting temperature for the shaft material than for the tip material ensures that the tip may be formed without any deformation or damage to the shaft.

Preferably, the catheter is at least partly coated with a hydrophilic surface coating, the coating covering at least a part of the tubular shaft and the tip, said hydrophilic surface coating exhibiting a low friction when wetted.

The hydrophilic surface coating preferably comprises a hydrophilic polymer. The hydrophilic polymer may be at least one of: polyvinyl compounds, polylactames, in particular such as polyvinyl pyrrolidones, polysaccharides, in particular heparin, dextran, xanthan gum, derivatised polysaccharides, hydroxy propyl cellulose, methyl cellulose, polyurethanes, polyacrylates, polyhydroxyacrylates, polymethacrylates, polyacrylamides, polyalkylene oxides, in particular polyethylene oxides, polyvinyl alcohols, polyamides, polyacrylic acid, copolymers of the previously mentioned polymers, copolymers of vinyl compounds and acrylates or anhydrides, copolymers of vinylpyrrolidone and hydroxy ethylmethyl acrylate, cationic copolymers of polyvinyl pyrrolidone and copolymer of polymethylvinyl ether and maleinic acid anyhydride, polyactide, polyethylene glycol and copolymers thereof. Preferably, the hydrophilic polymer is polyvinyl pyrrolidone.

The hydrophilic polymer may be attached to the substrate by means of a poly-urea network, or be cross-linked directly to the substrate. The cross-linking may be effected by means of irradiation, e.g. by electron beams or UV light.

In other embodiments, the entire catheter shaft and/or tip may be formed by a hydrophilic polymer, thereby providing a hydrophilic surface without the need for any additional coating.

It has been found by the same applicant that a tip having an outer diameter which at all places is equal to or lower than the outer diameter of the tubular shaft, such as being conically tapering in a forward, proximal direction, and being very soft, so that the hardness of the tip is equal to or lower than 60 micro Shore A, is remarkably easy to insert through a urethra, thereby providing much simplified handling. This was per se discussed in US 2016/184551. The soft tip automatically finds its way through the difficult passages of the urethra, and smoothly follows the bends, curves, restrictions and other difficult tracts, and without any risk of penetrating the urethra. The catheter tip adapt to the various anatomical situations as the insertion continues. The force necessary for pushing the catheter through the urethra is hereby also reduced. Thus, the present invention not only simplifies the handling of the catheter, but also significantly reduces the risk of injury. Consequently, this catheter is excellently suited for self-catheterization, and can safely and easily be used also by inexperienced users, and/or users suffering from poor dexterity.

Since a catheter with such a soft tip automatically guides it way into the urethra, a lower force during insertion is also required. The risk for penetration of the urethral wall is also lowered, which is of particularly useful for users having no or reduced sensitivity in the urethra tract.

The tip may be straight, extending in the same direction as the tubular shaft and forming a rounded forward end, thereby forming a Nelaton type catheter. However, preferably the tip is curved, forming a Tiemann or Coudé type catheter. Tiemann and Coudé catheters have a tip which is angled upward, to assist in negotiating the male prostatic curve. Thus, this tip form facilitates passage through the bladder neck in the presence of obstruction e.g. from a slightly enlarged prostate gland (e.g. in benign prostatic hyperplasia), and can be helpful for such and other difficult insertions.

The tip of the catheter forms a free forward end which presents the free forward end of the catheter body, which is spaced apart from the forward end portion of the tubular shaft. Thus, the shaft extends rearwardly from the rear end of the tip portion. A lumen extends through the tubular shaft, from the discharge end to the forward end of the catheter. The lumen ends in drainage openings, so-called eyes or eyelets. These eyes may be arranged in the forward end of the tubular shaft, in which case the lumen does not need to continue into the tip, which may as a consequence be solid. Alternatively, the eyes may be arranged in the tip, in which case the lumen at least partly continues into the tip.

The tip may advantageously have an E-modulus which is lower than the E-modulus of the tubular shaft, and preferably 10-30% lower.

The E-modulus of the tip is preferably within the range 6-16 MPa, and more preferably in the range 9-13 MPa.

In order to obtain good properties for handling, painless and easy insertion, etc, the material(s) of the tubular shaft and the tip are preferably prepared and composed in such a way that they fulfill at least some of the following requirements, and preferably essentially all of them:
- The material of the tubular shaft preferably has a hardness adequate for the intended use. Specifically, the micro Shore A hardness should preferably be in the range 75-95, and more preferably in the range 75-90, and most preferably within the range 78-85, for the tubular shaft.
- The material of the tip preferably has a lower hardness than the hardness of the tubular shaft. The micro Shore A hardness of the tip is equal to or lower than 60. Preferably, the micro Shore A hardness of the tip is in the range 30-50.
- It is further preferred that the materials of both the tip and the tubular shafts have melting temperatures exceeding 90 deg. C, and preferably exceeding 110 deg. C, and most preferably exceeding 130 deg. C.
- It is preferred that the materials are capable of being sterilized by known sterilization methods. In particular it is preferred that the materials have a radiation resistance such that it can endure at least 50 kGy essentially without degradation, in order to enable radiation sterilization of the urinary catheter.
- The material of the tubular shaft should preferably exhibit low resilience.
- The materials, and in particular the material of the tubular shaft, should preferably have good kinking properties.
- At least the material of the tip, and preferably also the material for the shaft, should be useable for molding, and in particular useable for injection molding.
- The materials should preferably be biocompatible.

The tip and the tubular shaft may be formed by the same material. The material of the tip and the tubular shaft may have the same or different properties. In case different properties are wanted, this may be accomplished with the use of the same material, e.g. by treatment of the materials in different ways. However, when different properties are wanted in the shaft and the tip, it is preferred to use different or at least partly different materials. For example, the tip can be made softer by using a different blend of polymers, by additives such as plasticizers, medical oil (i.e. oil of a medical grade), paraffin, etc.

The tubular shaft and the tip may be formed of a large variety of different substrate materials. However, preferably the tubular shaft and the tip are made of a polymer material. The tubular shaft and/or the tip may be formed by a polymer blend comprising primarily polyolefin, such as at least 80% by weight of polyolefin and therein possibly intermixed medical oil and/or paraffin. Polyolefin is a material comprising olefin monomers, such as one or several of ethylene, propylene, styrene, pentene, etc. The polyolefin may comprise at least one polymer selected from the group: polyethylene, polypropylene, and styrene block copolymer (SEBS). The polymer blend may further comprise a composition/polymer having molecules with active hydrogen(s), wherein the composition having molecules with active hydrogen(s) is preferably a polymer where the active hydrogen(s) is bound to the polymer via nitrogen.

Molecules with active hydrogen(s) are molecules having hydrogen that is prone to react with other substances, and thus to leave its position in the molecule. Examples of such compositions having molecules with active hydrogen groups are alcohols, amides, amines, urethane and acids. The composition having molecules with active hydrogen(s) is preferably at least one of polyamide and polyurethane. Preferably, the polymer blend comprises a weight percentage of the composition having molecules with active hydrogen(s) in the range of 2-20, and preferably in the range 3-15 and most preferably in the range 5-10. Such polymer blends are known from US 2012/0191073 and US 8168249 by the same applicant.

However, other materials may also be used, especially for the shaft, such as polyurethanes, latex rubbers, silicon rubbers, other rubbers, polyvinylchloride (PVC), other vinyl polymers, polyesters, polyacrylates, polyamides, polyolefines, thermoplastic elastomers, styrene block copolymers (SEBS), or polyether block amide (PEBA), and combinations of these.

For the injection molded tip, a thermoplastic material is preferably used. Suitable thermoplastic materials may be materials such as polyurethane, polyvinyl chloride, polyethylene, thermoplastic elastomers, such as olefin based thermoplastic elastomers, mixtures of thermoplastic elastomers and polyolefin(s), mixtures of such materials, and other thermo-formable materials. The use of thermoplastic materials means that the construction or the shape of the catheter may be partly or fully provided by treating the catheter or the catheter material with heat, such as melting or by solidifying the material by cooling.

The catheter is preferably arranged in a package, to maintain it sterile prior to use.

The catheter preferably has a radiation resistance such that it can endure at least 50 kGy essentially without degradation. Hereby, radiation sterilization of the medical device can be used, without affecting the properties of the medical device.

These and other aspects of the inventive concept will be apparent from and elicited with reference to the embodiments described hereinafter.

### Brief description of the drawings

By way of example embodiments of the invention will now be described with reference to the accompanying drawings in which:
Fig. 1 illustrates an embodiment of a catheter according to the invention;
Fig. 2 is a more detailed cross-sectional view of the catheter tip in the catheter of Fig. 1;
Fig. 3 is a cross-sectional view of a curved tip configuration, in accordance with another embodiment of the present invention;
Fig. 4 is a cross-sectional view of an overmolded tip of a catheter in accordance with an embodiment of the present invention;
Fig. 5 is a cross-sectional view of a catheter with a molded tip in accordance with another embodiment of the present invention;
Fig. 6 is a cross-sectional view of an injection molding apparatus for forming an injection molded tip in accordance with an embodiment of the present invention; and
Fig. 7 is a cross-sectional view of an injection molding apparatus for forming an injection molded tip in accordance with another embodiment of the present invention.

### Description of preferred embodiments

In the following detailed description preferred embodiments of the invention will be described. However, it is to be understood that features of the different embodiments are exchangeable between the embodiments and may be combined in different ways, unless anything else is specifically indicated. It may also be noted that, for the sake of clarity, the dimensions of certain components illustrated in the drawings may differ from the corresponding dimensions in real-life implementations. Even though in the following description, numerous specific details are set forth to provide a more thorough understanding of the present invention, it will be apparent to one skilled in the art that the present invention may be practiced without these specific details. In other instances, well known constructions or functions are not described in detail, so as not to obscure the present invention.

The following discussion is in particular concerned with hydrophilic urinary catheters for intermittent use. However, the invention can also be used in relation to other types of urinary catheters.

In the following, embodiments of a catheter according to the invention, and producible by methods in accordance with embodiments of the invention, will first be discussed, and thereafter embodiments of methods in accordance with the invention.

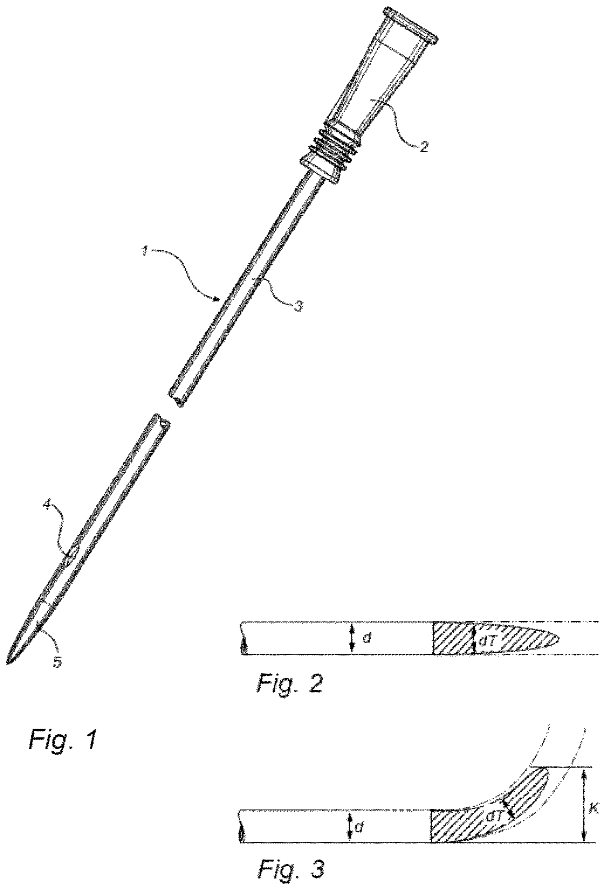

A catheter 1 as illustrated in Fig. 1, comprises a flared rearward portion 2 and an elongate shaft or tube 3 projecting forwardly from the rearward portion 2. An open-ended internal lumen extends from the rear end of the rearward portion 2 to one or more drainage apertures 4 in a forward end of the catheter. At the forward end of the elongate shaft 3, a tip 5 is arranged, having a rounded tip end. The tip may have a tapering form, as in the illustrative example, but other shapes are also feasible, as will be discussed in more detail in the following. The rearward portion 2 may function as a connector of the catheter 1, being connectable to other devices, such as a urine collection bag, a drainage tube or the like.

The connector, i.e. the flared rearward portion 2 is optional, and catheters without any flared rearward portion may also be used. In case a flared connector is used, this may be formed integrally and monolithically at the rearward end of the tubular shaft. However, it may also be formed as a separate component, being connected to the tubular shaft by means of welding, adhesion or the like. It may also be injection molded directly in place. In this case, the injection molding of the tip at the forward end of the tubular shaft and the connector at the rearward end of the tubular shaft can be performed simultaneously.

The drainage openings are arranged in the forward end of the tubular shaft, so that the lumen does not need to continue into the tip, which may as a consequence be solid.

At least a part of the elongate tube 3 forms an insertable length to be inserted through a body opening of the user, such as the urethra in case of a urinary catheter. By insertable length is normally meant that length of the elongate tube 2 which is insertable into the urethra of the patient during ordinary use. In case a hydrophilic catheter is used, the insertable length is coated with a hydrophilic material, for example PVP, or is made of hydrophilic material. Typically, the insertable length is 80-140 mm for a female patient and 200-350 mm for a male patient.

The tip may be straight, extending in the same direction as the tubular shaft and forming a rounded forward end, thereby forming a Nelaton type catheter. Such an embodiment is illustrated in Figs. 1 and 2. The tip has an outer diameter dT which at all places is equal to or lower than the outer diameter d of the tubular shaft. The tip is preferably arranged conically tapering in the forward direction, to end in a rounded tip. However, alternative configurations are also feasible. For example, the tip may over part of its extension have a cylindrical configuration with the same diameter, may be arranged in a stepwise reduced diameter, may have parts being slightly enlarged, such as a ball shaped forward end, or the like. For example, the end may be provided with a slightly enlarged rounded or ball-shaped head portion, which, however, may have at least a slightly smaller diameter than the diameter of the tube, or a diameter slightly or significantly greater than the diameter of the tube.

The tip may also be curved, forming a Tiemann or Coudé type catheter. Such a tip is illustrated in Fig. 3. The curved tip also has a diameter dT which at all places is equal to or lower than the outer diameter d of the tubular shaft. The curved tip preferably has a height K in a lateral direction which is higher than the diameter d of the tube, and preferably the relation K/d is in the range 1.5 - 2.

The tip of the catheter forms a free forward end which presents the free forward end of the catheter body, which is spaced apart from the forward end portion of the tubular shaft. Thus, the shaft extends rearwardly from the rear end of the tip portion. A lumen extends through the tubular shaft, from the discharge end to the forward end of the catheter. The lumen ends in drainage openings, so-called eyes, These eyes may be arranged in the forward end of the tubular shaft, in which case the lumen does not need to continue into the tip, which may as a consequence be solid.

The tip may have an outer diameter which at all places is equal to or lower than the outer diameter of the tubular shaft. However, alternatively, the tip may have a diameter which at least on some positions has a diameter exceeding the diameter of the shaft. For example, the tip may form a ball shape at the end of the catheter.

The tips may be designed, formed and connected to the tubular shaft in various ways, and some exemplary embodiments of this will now be discussed in more detail.

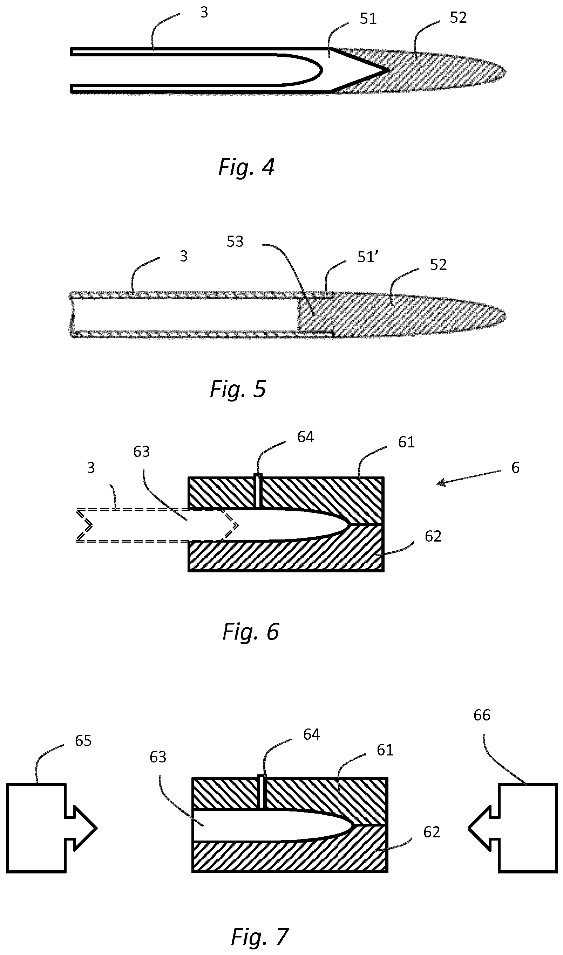

According to one embodiment for forming a urinary catheter, and in particular a urinary catheter tip, schematically illustrated in Fig. 4, the method comprises a first step of providing a tubular catheter shaft 3. The catheter shaft may e.g. be obtained by cutting a suitable length from a continuous extruded tube.

In a second step, a connector is arranged at one end of the catheter shaft. The connector may e.g. be made separately, and be connected to the catheter shaft by welding, adhesion or the like. However, alternatively, the connector may be formed directly from the shaft material, e.g. by thermoforming, to be integrally and monolithically connected to the shaft. In some embodiments, the connector may also be omitted.

In a further step, the insertion end of the catheter shaft, i.e. the end being opposite to the connector end, is thermoformed into a closed end 51. Such thermoforming of a closed end is per se well known in the art, and may e.g. by obtained by inserting the non-closed end into a heated die, e.g. forming a tapering and/or rounded tip.

In a further step, the thermoformed, closed end of the catheter shaft is inserted into an injection mold cavity, as will be discussed in more detail below, and a thermoplastic polymer material is injected into the mold cavity to form a tip 52 on the thermoformed, closed end 51 of the tubular catheter shaft. Hereby, the thermoformed, closed end of the catheter shaft is over molded by the tip in the tip forming injection molding step.

The steps may be performed in a different order. For example, the (optional) connector may be connected or formed to the catheter shaft before or after the injection molding of the tip.

The thermoformed end 51 preferably forms a tapered contacting surfaces for the tip 52. Hereby, a larger attachment area, and a stronger attachment, is obtained.

In another embodiment for forming a urinary catheter, and in particular a urinary catheter tip, shown schematically in Fig. 5, the method comprises a first step of providing a tubular catheter shaft 3. The catheter shaft may e.g. be obtained by cutting a suitable length from a continuous extruded tube.

In a second step, a connector is arranged at one end of the catheter shaft. The connector may e.g. be made separately, and be connected to the catheter shaft by welding, adhesion or the like. However, alternatively, the connector may be formed directly from the shaft material, e.g. by thermoforming, to be integrally and monolithically connected to the shaft. In some embodiments, the connector may also be omitted.

In a further step, the non-closed end 51' of the catheter shaft is inserted into an injection mold cavity (to be discussed in further detail in the following), and a thermoplastic polymer material is injected into the mold cavity to form a tip 52' on the non-closed end 51' of the tubular catheter shaft. Hereby, the tip material enters into the lumen of the catheter shaft to a controlled degree. To this end, the injecting of the thermoplastic polymer material into the mold cavity is controlled so that a limited amount of thermoplastic polymer enters into the lumen. How far the injection molded material penetrates into the lumen of the catheter shaft may be controlled by controlling the injection molding parameters, such as one or several of injection speed, injection time, the melt temperature, and volume of injected material. Hereby, the injected material forms a controlled stub shaft 53 protruding into the opening and lumen of the non-closed end 51', and with the rest of the tip 52' extending out from the catheter shaft in a proximal, forward direction. Hereby, a strong bond to the shaft is formed.

Also in this embodiment the steps may be performed in a different order. For example, the (optional) connector may be connected or formed to the catheter shaft before or after the injection molding of the tip.

In order to provide a stronger connection between the tip and the tubular shaft, the inner surface of the tubular shaft may be provided with a surface texture or surface features, e.g. to form a mechanical engagement. For example, the tubular shaft may, on an interior wall of the lumen and closed to the end, be provided with indent elements, such as holes, grooves or the like.

With reference to Fig. 6, the mold apparatus may comprise a mold 6, forming a mold cavity therein. The mold may comprise two separable parts 61, 62, which are brought together to form the mold cavity, and which may be separated for extraction of the catheter when the tip has been formed. However, alternatively, the mold may be formed by a single part. The mold cavity further comprises an open end 63, through which the catheter shaft 3, illustrated with dashed lines, may be inserted. The cavity opening 63 is preferably dimensioned to snuggly received the catheter shaft, so that the internal diameter of the opening is essentially equal to, or only slightly larger, than the outer diameter of the catheter shaft. The mold cavity is shaped in correspondence with the shape of the tip to be formed. A gate 64 is provided to allow melted, liquefied material to be inserted into the mold cavity. The gate may e.g. be positioned in one of the parts 61, 62, and relatively close to the end of the inserted catheter shaft. However, other positions are also feasible, such as in a centerline of the catheter shaft, in front of the forward most part of the tip to be formed, i.e. at the apex of the tip. Multiple gates may also be used. A small depression can be designed into the internal end of the gate, so that any gate residue in the formed part is not protruding from the surface of the tip.

The mold may be non-heated, whereby the only heat is provided by the melted, liquefied material injected through the gate. However, alternatively, the mold may be heated. A mold may be heated in various ways, such as by arranging an internal heater within the mold, or by an external heater. In the illustrative example of Fig. 7, an external heater 66 is schematically illustrated. The heater may heat the mold by irradiation, by hot air, etc, as is per se known in the art.

When the inserted catheter shaft presents a closed end, the end of the catheter shaft serves as a stop for the material injected into the mold cavity. Thus, in this case, the inserted catheter end may be seen as forming one of the walls of the mold cavity. This ensures that the mold cavity will fill properly, resulting in a well formed tip of controlled geometry.

When a catheter shaft with a non-closed end is inserted into the mold cavity, there is no such stop. However, by adequate control of the injection molding, the degree of penetration into the lumen of the catheter shaft of the injected material may be controlled, so that a suitable stub-shaft is formed.

However, to further control the degree of penetration of the injected material into the lumen, a controllable counter pressure may also be provided, by a pressure device 65. This may e.g. be provided through the lumen of the catheter shaft. Such an overpressure may then be controlled to provide a resistance for the thermoplastic polymer material to enter into the lumen, and the overpressure may be controlled to provide an adequate length of penetration. Providing a higher overpressure will provide a stronger resistance and a lower depth of penetration, whereas a lower overpressure, or no overpressure at all, will provide less resistance and a corresponding deeper penetration.

In both the above-discussed methods, a very strong bond between the shaft and the injection molded tip is created, inter alia due to the large overlapping areas.

The mold cavity may further be heated during molding, whereby the material of the shaft will be softened not only by the heated injection molded thermoplastic polymer material, but also by the heating of the mold. This ensures an even stronger attachment of the tip to the shaft, since the materials will hereby also be fused together. However, alternatively the heat provided by the heated thermoplastic polymer that is injected is in itself sufficient to provide adequate heating to form a strong attachment, in which case no additional heating of the mold is necessary.

Preferably, the thermoplastic polymer to be injected is heated to a temperature exceeding the melting temperature of the tubular catheter shaft immediately prior to being injection molded in to the mold cavity, and preferably exceeding said melting temperature with at least 10 degrees C. Hereby, it has been found, a very strong adhesion between the shaft and the tip is obtained, without any risk of damage and deformation of the shaft.

The material for injection molding is preferably one that is compatible with the catheter shaft material, to ensure good bonding between the molded tip and the catheter shaft.

After the injected tip material cools, optionally the mold halves are separated and the catheter shaft and tip are removed from the cavity. Alternatively, the mold may be one piece, and the catheter shaft with the tip may just be pulled free of the mold. The result is a formed tip of desired geometry that is integral with the end of the catheter shaft.

In these methods, the forming of the tip may further comprise cooling of the mold cavity to solidify the injected thermoplastic polymer material, before removing the tubular catheter shaft with the formed tip from the mold. Cooling of the mold, after having introduced the thermoplastic polymer material, and thereby having formed the tip, ensures the integrity of the shaped tip, and also facilitates removal from the mold cavity.

The methods may further comprise the step of inserting an elongate insert into the axially extending lumen prior to inserting an end of the catheter shaft into the mold, wherein the insert has a smaller outer diameter than the inner diameter of the lumen, forming a gap between an outer circumferential surface of the elongate insert and an inner circumferential surface of the lumen, the insert thereby being loosely arranged within the lumen. The elongate insert may e.g. be a narrow steel rod or the like. Insertion of such an elongate insert does not affect the penetration of the injected thermoplastic polymer material, but ensures that the shape of the catheter shaft is not altered during the injection molding process. For example, it prevents inadvertent collapsing of the shaft. Thus, the elongate insert ensures that the shaft remain straight, and with an equally sized lumen along its length. However, in many cases, depending on the materials used and the process parameters, such as the temperature, there is no need for such an additional precaution, and in such cases there is no need for use of such an elongate insert.

The catheter can be non-coated, and can e.g. be used together with a gel lubricant or the like for insertion. The catheter may also be formed by a material having low friction, and can e.g. be made by a hydrophilic material. However, the catheter is preferably coated, as have been discussed in the foregoing. In particular, for catheters, it is preferred to coat the outer surface, at least of the insertable part, with a hydrophilic coating. Many different types of well-known hydrophilic surfaces can be used.

In case a hydrophilic coating is used, it is preferred that both the tip and an insertable part of the tubular shaft are provided with said coating. The coating may be applied after joining of the tip and the tubular shaft, but may alternatively be provided separately to the tip and the tubular shaft, prior to joining.

Upon use, the catheter, when being provided with a hydrophilic coating, or being made by a hydrophilic material, is wetted by a wetting fluid, whereby the hydrophilic surface becomes slippery and easy to insert into e.g. the urethra of the patient, i.e. to provide a low-friction character of the surface. The wetting fluid is preferably a water-based liquid, i.e. using water as a solvent.

The person skilled in the art realizes that the present invention by no means is limited to the preferred embodiments described above. On the contrary, many modifications and variations are possible within the scope of the appended claims. For instance, the tip need not be continuously tapering, but have other geometrical shapes. Further, the tip may be either straight, pointing directly in the longitudinal direction of the catheter, or be slightly curved, so that the end of the tip points in a direction which is non-parallel to the longitudinal direction of the catheter. Further, many different materials and material combinations may be used to produce the tubular shaft and the tip, and still obtain the desired material properties. Still further, the access openings/drainage eyes may be provided in either the tip or in the forward end of the tubular shaft, or even in both. Such and other modifications should be construed to fall within the scope of the appended claims.

## Claims

1. A method for producing a urinary catheter (1), comprising the steps:
providing a tubular catheter shaft (3) having an axially extending lumen therein;
inserting an end of the tubular catheter shaft with a non-closed opening into said lumen into an injection mold cavity; and
injecting thermoplastic polymer material into the mold cavity to form a tip (52) on the end of the tubular catheter shaft (3), wherein the injecting is controlled so that a limited amount of thermoplastic polymer enters into the lumen.

2. The method of claim 1, further comprising providing an overpressure within the axially extending lumen during the injecting of the thermoplastic polymer material into the mold cavity.

3. The method of any one of the preceding claims, wherein the forming of the tip further comprises cooling the mold cavity to solidify the injected thermoplastic polymer material, and removing the tubular catheter shaft (3) with the formed tip (52; 52') from the mold.

4. The method of any one of the preceding claims, further comprising the step of coating at least a part of the tubular shaft and the tip with a hydrophilic coating.

5. The method of any one of the preceding claims, further comprising the step of inserting an elongate insert into the axially extending lumen prior to inserting an end of the catheter shaft into the mold, wherein the insert has a smaller outer diameter than the inner diameter of the lumen, forming a gap between an outer circumferential surface of the elongate insert and an inner circumferential surface of the lumen, the insert thereby being loosely arranged within the lumen.

6. The method of any one of the preceding claims, wherein the thermoplastic polymer is heated to a temperature exceeding the melting temperature of the tubular catheter shaft immediately prior to being injection molded into the mold cavity, and preferably exceeding said melting temperature with at least 10 degrees C.

7. A urinary catheter comprising a tubular shaft (3) extending between an insertable end and a discharge end, **characterized by** the discharge end being thermoformed to form a closed end (51), and a tip (52) fixedly connected to said closed end of the tubular shaft (3).

8. The urinary catheter of claim 7, wherein the hardness of the tip (52) is equal to or lower than 60 micro Shore A, and preferably equal to or lower than 50 micro Shore A, and most preferably equal to or lower than 45.

9. The urinary catheter of any one of the claims 7-8, wherein the tip (52) has a lower micro Shore A hardness than the tubular shaft (3), and preferably a micro Shore A hardness that is at least 10% lower, and more preferred at least 30% lower, and most preferably at least 50% lower.

10. The urinary catheter of any one of the claims 7-9, wherein the thermoformed end (51) forms a tapered contacting surface for the tip.

11. The urinary catheter of any one of the claims 7-10, wherein the tip (52) and the tubular shaft (3) are formed of different materials.

12. The urinary catheter of claim 11, wherein a difference between a melting temperature of the material of the tip (52) and a melting temperature of the material of the tubular shaft (3) is less than 20 degrees C, and preferably less than 15 degree C, and most preferably less than 10 degree C.

13. The urinary catheter of claim 11 or 12, wherein the melting temperature of the material of the tubular shaft (3) is higher than the melting temperature of the material of the tip (52).

14. The urinary catheter of any one of the claims 7-13, wherein the catheter (1) is at least partly coated with a hydrophilic surface coating, the coating covering at least a part of the tubular shaft and the tip, said hydrophilic surface coating exhibiting a low friction when wetted.

## Patentansprüche

1. Verfahren zur Herstellung eines Harnkatheters (1), umfassend die folgenden Schritte:
Bereitstellen eines rohrförmigen Katheterschafts (3), der einen sich axial erstreckenden Lumen darin aufweist;
Einführen eines Endes des rohrförmigen Katheterschafts mit einer nicht geschlossenen Öffnung in das Lumen in einen Spritzgussformhohlraum; und
Einspritzen von thermoplastischem Polymermaterial in den Formhohlraum, um eine Spitze (52) an dem Ende des rohrförmigen Katheterschafts (3) auszubilden, wobei das Einspritzen so gesteuert wird, dass eine begrenzte Menge an thermoplastischem Polymer in das Lumen eintritt.

2. Verfahren nach Anspruch 1, ferner umfassend das Bereitstellen eines Überdrucks innerhalb des sich axial erstreckenden Lumens während des Einspritzens des thermoplastischen Polymermaterials in den Formhohlraum.

3. Verfahren nach einem der vorhergehenden Ansprüche, wobei das Ausbilden der Spitze ferner Kühlen des Formhohlraums, um das eingespritzte thermoplastische Polymermaterial zu verfestigen, und Entfernen des rohrförmigen Katheterschafts (3) mit der ausgebildeten Spitze (52; 52') aus der Form umfasst.

4. Verfahren nach einem der vorhergehenden Ansprüche, ferner umfassend den Schritt des Beschichtens von mindestens einem Teil des rohrförmigen Schafts und der Spitze mit einer hydrophilen Beschichtung.

5. Verfahren nach einem der vorhergehenden Ansprüche, ferner umfassend den Schritt des Einführens eines länglichen Einsatzes in das sich axial erstreckende Lumen vor dem Einführen eines Endes des Katheterschafts in die Form, wobei der Einsatz einen kleineren Außendurchmesser als der Innendurchmesser des Lumens aufweist, wobei ein Spalt zwischen einer Außenumfangsfläche des länglichen Einsatzes und einer Innenumfangsfläche des Lumens ausgebildet wird, wobei der Einsatz dadurch lose innerhalb des Lumens angeordnet ist.

6. Verfahren nach einem der vorhergehenden Ansprüche, wobei das thermoplastische Polymer auf eine Temperatur erhitzt wird, die die Schmelztemperatur des rohrförmigen Katheterschafts unmittelbar vor dem Spritzgießen in den Formhohlraum übersteigt und bevorzugt die Schmelztemperatur um mindestens 10 Grad C übersteigt.

7. Harnkatheter, umfassend einen rohrförmigen Schaft (3), der sich zwischen einem einführbaren Ende und einem Auslassende erstreckt, **dadurch gekennzeichnet, dass** das Auslassende thermogeformt ist, um ein geschlossenes Ende (51) auszubilden, und eine Spitze (52) fest mit dem geschlossenen Ende des rohrförmigen Schafts (3) verbunden ist.

8. Harnkatheter nach Anspruch 7, wobei die Härte der Spitze (52) gleich oder niedriger als 60 Mikro-Shore A und bevorzugt gleich oder niedriger als 50 Mikro-Shore A und am meisten bevorzugt gleich oder niedriger als 45 ist.

9. Harnkatheter nach einem der Ansprüche 7-8, wobei die Spitze (52) eine niedrigere Mikro-Shore-A-Härte als der rohrförmige Schaft (3) und bevorzugt eine Mikro-Shore-A-Härte aufweist, die mindestens 10 % niedriger und bevorzugter mindestens 30 % niedriger und am meisten bevorzugt mindestens 50 % niedriger ist.

10. Harnkatheter nach einem der Ansprüche 7-9, wobei das thermogeformte Ende (51) eine sich verjüngende Kontaktfläche für die Spitze ausbildet.

11. Harnkatheter nach einem der Ansprüche 7-10, wobei die Spitze (52) und der rohrförmige Schaft (3) aus unterschiedlichen Materialien ausgebildet sind.

12. Harnkatheter nach Anspruch 11, wobei eine Differenz zwischen einer Schmelztemperatur des Materials der Spitze (52) und einer Schmelztemperatur des Materials des rohrförmigen Schafts (3) weniger als 20 Grad C und bevorzugt weniger als 15 Grad C und am meisten bevorzugt weniger als 10 Grad C beträgt.

13. Harnkatheter nach Anspruch 11 oder 12, wobei die Schmelztemperatur des Materials des rohrförmigen Schafts (3) höher als die Schmelztemperatur des Materials der Spitze (52) ist.

14. Harnkatheter nach einem der Ansprüche 7-13, wobei der Katheter (1) mindestens teilweise mit einer hydrophilen Oberflächenbeschichtung beschichtet ist, wobei die Beschichtung mindestens einen Teil des rohrförmigen Schafts und der Spitze bedeckt, wobei die hydrophile Oberflächenbeschichtung eine geringe Reibung zeigt, wenn sie benetzt ist.

## Revendications

1. Procédé permettant la production d'un cathéter urinaire (1), comprenant les étapes : fourniture d'une tige (3) de cathéter tubulaire comportant une lumière s'étendant axialement dans celle-ci ; insertion d'une extrémité de la tige de cathéter tubulaire avec une ouverture non fermée dans ladite lumière dans une cavité de moule d'injection ; et
injection d'un matériau polymère thermoplastique dans la cavité de moule pour former une pointe (52) sur l'extrémité de la tige (3) de cathéter tubulaire, ladite injection étant commandée afin qu'une quantité limitée de polymère thermoplastique entre dans la lumière.

2. Procédé de la revendication 1, comprenant en outre la fourniture d'une surpression à l'intérieur de la lumière s'étendant axialement durant l'injection du matériau polymère thermoplastique dans la cavité de moule.

3. Procédé de l'une quelconque des revendications précédentes, ladite formation de la pointe comprenant en outre un refroidissement de la cavité de moule pour solidifier le matériau polymère thermoplastique injecté, et un retrait de la tige (3) de cathéter tubulaire avec la pointe formée (52 ; 52') du moule.

4. Procédé de l'une quelconque des revendications précédentes, comprenant en outre l'étape de revêtement d'au moins une partie de la tige tubulaire et de la pointe avec un revêtement hydrophile.

5. Procédé de l'une quelconque des revendications précédentes, comprenant en outre l'étape d'insertion d'un insert allongé dans la lumière s'étendant axialement avant d'insérer une extrémité de la tige de cathéter dans le moule, ledit insert possédant un diamètre externe inférieur au diamètre interne de la lumière, formant un espace entre une surface circonférentielle externe de l'insert allongé et une surface circonférentielle interne de la lumière, l'insert étant ainsi agencé de manière lâche à l'intérieur de la lumière.

6. Procédé de l'une quelconque des revendications précédentes, ledit polymère thermoplastique étant chauffé à une température dépassant la température de fusion de la tige de cathéter tubulaire immédiatement avant d'être moulé par injection dans la cavité de moule, et de préférence dépassant ladite température de fusion d'au moins 10 degrés C.

7. Cathéter urinaire comprenant une tige tubulaire (3) s'étendant entre une extrémité insérable et une extrémité de décharge, **caractérisé par** l'extrémité de décharge qui est thermoformée pour former une extrémité fermée (51), et une pointe (52) fixement raccordée à ladite extrémité fermée de la tige tubulaire (3).

8. Cathéter urinaire de la revendication 7, ladite pointe (52) possédant une dureté égale ou inférieure à 60 micro Shore A, et de préférence inférieure ou égale à 50 micro Shore A, et idéalement inférieure ou égale à 45.

9. Cathéter urinaire de l'une quelconque des revendications 7-8, ladite pointe (52) possédant une dureté micro Shore A inférieure à celle de la tige tubulaire (3), et de préférence une dureté micro Shore A qui est inférieure d'au moins 10 %, et mieux encore d'au moins 30 %, et idéalement d'au moins 50 %.

10. Cathéter urinaire de l'une quelconque des revendications 7-9, ladite extrémité thermoformée (51) formant une surface de contact conique pour la pointe.

11. Cathéter urinaire de l'une quelconque des revendications 7-10, ladite pointe (52) et ladite tige tubulaire (3) étant formées de matériaux différents.

12. Cathéter urinaire de la revendication 11, une différence entre une température de fusion du matériau de la pointe (52) et une température de fusion du matériau de la tige tubulaire (3) étant inférieure à 20 degrés C, et de préférence inférieure à 15 degrés C, et idéalement inférieure à 10 degrés C.

13. Cathéter urinaire de la revendication 11 ou 12, ladite température de fusion du matériau de la tige tubulaire (3) étant supérieure à la température de fusion du matériau de la pointe (52).

14. Cathéter urinaire de l'une quelconque des revendications 7-13, ledit cathéter (1) étant au moins partiellement revêtu d'un revêtement de surface hydrophile, le revêtement couvrant au moins une partie de la tige tubulaire et de la pointe, ledit revêtement de surface hydrophile présentant un faible frottement lorsqu'il est mouillé.
